# EUROPÄISCHE PATENTSCHRIFT

(11) **EP 2 853 187 B1**
(45) Veröffentlichungstag und Bekanntmachung des Hinweises auf die Patenterteilung: **31.10.2018**
(21) Anmeldenummer: 14185965.2
(22) Anmeldetag: 23.09.2014
(51) Int. Cl.: A61B 1/00, A61B 1/07, G02B 23/24, G02B 23/26, G02B 6/04, G02B 6/255

(54) **Herstellen eines Endoskops mit einer Lichtleiteinrichtung**
Manufacture of an endoscope with a light conducting device
Fabrication d'un endoscope doté d'un dispositif de conducteur lumineux

(30) Priorität: 27.09.2013 DE 102013110692
(43) Veröffentlichungstag der Anmeldung: 01.04.2015
(73) Patentinhaber: Karl Storz SE & Co. KG, 78532 Tuttlingen (DE)
(72) Erfinder: Eisenkolb, Peter, 78532 Tuttlingen (DE)

(56) Entgegenhaltungen:
- EP-A2- 2 515 154
- DE-A1-102011 007 878
- US-A1- 2004 105 649
- US-A1- 2008 260 335

## Beschreibung

Die vorliegende Erfindung ist auf ein Endoskop mit Lichtleitfasern zum Leiten von Beleuchtungslicht zum distalen Ende des Endoskops und auf Verfahren zum Herstellen eines Endoskops bezogen.

In der medizinischen und technischen Endoskopie ist in der Regel eine Beleuchtung des betrachteten Objekts erforderlich. Zur Erzeugung von Beleuchtungslicht mit einem hohen Lichtstrom werden oft separate oder in das proximale Ende des Endoskops integrierte Lichtquelleneinrichtungen verwendet. Vom proximalen Ende zum distalen Ende des Endoskops wird das Beleuchtungslicht mittels eines oder mehrerer Bündel von Lichtleitfasern übertragen.

Es gibt eine deutliche Tendenz, die Schäfte von Endoskopen immer dünner auszuführen. Dabei wird der für die Lichtleitfasern zur Verfügung stehende Bauraum immer kleiner, und der Aufwand in Konstruktion und Fertigung steigt. Herkömmliche Konstruktionen und Fertigungsverfahren wie beispielsweise in der Druckschrift DE 10 2011 007878 A1 offenbart, müssen in Frage gestellt und modifiziert oder durch neue ersetzt werden.

Eine Aufgabe der vorliegenden Erfindung besteht darin, ein verbessertes Verfahren zum Herstellen eines Endoskops und ein verbessertes Endoskop zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, beim Herstellen eines Endoskops ein Lumen, in dem Lichtleitfasern angeordnet sind, zu verengen, während die Lichtleitfasern auf eine Temperatur erwärmt sind, bei der sie plastisch verformbar sind. Durch das Verengen des Lumens werden die Lichtleitfasern miteinander verschmolzen bzw. verschweißt. Dabei werden die Lichtleitfasern insbesondere gleichzeitig mit der Wand oder den Wänden, die das Lumen begrenzen, verschweißt. Die Querschnitte der einzelnen Lichtleitfasern werden verändert und die Zwischenräume zwischen den einzelnen Lichtleitfasern verkleinert. Im Idealfall entsteht innerhalb des verengten Lumens ein dichtes Gefüge von Lichtleitfasern mit jeweils sechseckigem Querschnitt in einer bienenwabenförmigen Anordnung.

Bei einem Verfahren zum Herstellen eines Endoskops werden Lichtleitfasern in ein Lumen in dem Endoskop eingebracht, die Lichtleitfasern erwärmt, das Lumen verengt und die Lichtleitfasern verschmolzen, wobei das Verengen des Lumens und das Verschmelzen der Lichtleitfasern zumindest teilweise gleichzeitig erfolgen.

Das Lumen weist insbesondere einen kreisförmigen Querschnitt oder einen kreisringförmigen Querschnitt oder einen Querschnitt, dessen Rand im Wesentlichen aus zwei Kreisbogenabschnitten mit gleichen oder unterschiedlichen Radien besteht, oder einen Querschnitt, dessen Rand zwei oder mehr kreisbogenförmige Abschnitte mit unterschiedlichen oder gleichen Radien umfasst, oder einen anderen Querschnitt auf. Der Querschnitt des Lumens nach dem Verengen ist insbesondere dem Querschnitt des Lumens vor dem Verengen geometrisch ähnlich oder im Wesentlichen geometrisch ähnlich. Insbesondere weist das Lumen einen geraden oder gekrümmten, langgestreckten und schmalen Querschnitt mit einer vom einen Ende zum anderen Ende hin kontinuierlich variierenden Breite auf, wobei beim Verengen des Lumens vor allem die Breite des Querschnitts des Lumens reduziert wird.

Die Lichtleitfasern werden insbesondere zusammen mit der das Lumen begrenzenden Wand oder zusammen mit den das Lumen begrenzenden Wänden erwärmt. Die Lichtleitfasern werden insbesondere durch Wärmeleitung ausgehend von einer das Lumen begrenzenden Wand erwärmt, wobei die Wand durch Wärmeleitung, Wärmestrahlung oder auf andere Weise erwärmt werden kann. Die Lichtleitfasern weisen insbesondere ein anorganisch-nichtmetallisches Glas, ein anderes anorganisches Glas, ein anderes amorphes Material, ein Polymer oder einen anderen Kunststoff auf. Die Lichtleitfasern werden insbesondere bis zu einer Transformationstemperatur oder Glastemperatur oder Glasübergangstemperatur erwärmt, bei der das Material der Lichtleitfasern eine gummiartig bis zähflüssige Konsistenz und/oder eine ausreichende Duktilität aufweist, um plastisch verformt zu werden. Das Verschmelzen der Lichtleitfasern wird insbesondere durch das Verengen des Lumens und den resultierenden mechanischen Druck bewirkt, kann jedoch, insbesondere aufgrund von Benetzungseigenschaften des Materials der Lichtleitfasern schon während oder nach dem Erwärmen und vor dem Verengen des Lumens beginnen.

Mit dem Verfahren kann ein mechanisch robustes Gefüge von Lichtleitfasern in einem Lumen in einem Endoskop geschaffen werden. Das Gefüge der Lichtleitfasern weist insbesondere nur noch kleine oder gar keine Hohlräume mehr auf. Kleine verbleibende Hohlräume können danach durch ein Material aufgefüllt werden, das bei niedrigeren Temperaturen schmilzt als das Material der Lichtleitfasern. Damit kann ein für Wasserdampf und/oder andere Fluide undurchlässige Struktur geschaffen werden, die insbesondere zum hermetisch dichten Abschluss eines Volumens innerhalb des Endoskops geeignet sein kann.

Bei einem Verfahren, wie es hier beschrieben ist, finden das Verengen des Lumens und das Verschmelzen der Lichtleitfasern insbesondere am distalen Ende des Endoskops statt.

Bei einem Verfahren, wie es hier beschrieben ist, wird insbesondere eine Lichteintrittsfläche oder eine Lichtaustrittsfläche an den Lichtleitfasern in oder an dem verschmolzenen Bereich der Lichtleitfasern gebildet.

Die Bildung einer unmittelbar oder optional nach Auffüllen von verbliebenen kleinen Hohlräumen fluiddichten Struktur von Lichtleitfasern gemäß einem Verfahren, wie es hier beschrieben ist, ermöglicht insbesondere die Bildung einer Lichtaustrittsfläche am distalen Ende des Endoskops. Dazu werden die Lichtleitfasern nach dem Erwärmen, Verengen und Verschmelzen insbesondere außerhalb des Endoskops abgeschnitten und die Schnittfläche poliert und optional entspiegelt und/oder auf andere Weise vergütet. Dadurch kann ohne ein weiteres transparentes Fensterbauteil oder ein anderes Bauteil unmittelbar aus den Lichtleitfasern eine Lichtaustrittsfläche geschaffen werden, die gleichzeitig das Endoskop hermetisch dicht verschließt und auch beim Autoklavieren ein Eindringen von Feuchtigkeit und/oder anderen Fluiden verhindert.

Zusätzlich ist das Verfahren auch am proximalen Ende des Endoskops verwendbar, insbesondere zur Bildung einer Lichteintrittsfläche für Beleuchtungslicht an einer Kupplungsstelle, an der das Endoskop mittels eines Lichtleitkabels mit einer separaten Lichtquelle koppelbar ist. Auch am proximalen Ende eines Endoskops kann das Verfahren so ausgeführt werden, dass ohne Verwendung eines transparenten Fensterbauteils oder eines anderen zusätzlichen Bauteils eine gegen das Eindringen von Wasserdampf oder anderen Fluiden hermetisch dichte Lichteintrittsfläche entsteht.

Bei einem Verfahren, wie es hier beschrieben ist, werden insbesondere Lichtleitfasern mit einer das Lumen begrenzenden Wand verschweißt.

Die Lichtleitfasern werden insbesondere beim Verengen des Lumens mit einer oder mehreren das Lumen begrenzenden Wänden verschweißt. Die das Lumen begrenzende Wand oder die das Lumen begrenzenden Wände weisen insbesondere einen chirurgischen Edelstall oder ein anderes metallisches Material auf. Das Verschweißen bzw. stoffschlüssige Fügen der Lichtleitfasern mit der Wand oder den Wänden, die das Lumen begrenzen, kann die mechanische Robustheit erhöhen und dazu beitragen, dass keine Fluide durch das verengte Lumen hindurch gelangen können.

Bei einem Verfahren, wie es hier beschrieben ist, werden die Lichtleitfasern insbesondere mit einem Außenschaft des Endoskops verschweißt.

Bei einem Verfahren, wie es hier beschrieben ist, werden die Lichtleitfasern insbesondere mit einem Innenschaft des Endoskops verschweißt.

Der Außenschaft weist insbesondere chirurgischen Edelstahl oder ein anderes metallisches Material auf. Der Außenschaft weist insbesondere vor dem Verengen des Lumens die Gestalt einer Mantelfläche oder eines Ausschnitts einer Mantelfläche eines Kreiszylinders auf. Der Außenschaft bildet insbesondere einen großen Teil oder den weit überwiegenden Teil der äußeren Oberfläche des Endoskops.

Der Innenschaft weist insbesondere einen chirurgischen Edelstahl oder ein anderes metallisches Material auf. Der Innenschaft weist insbesondere die Gestalt einer Mantelfläche oder eines Ausschnitts einer Mantelfläche eines Kreiszylinders auf. Das Lumen des Innenschafts ist insbesondere zur Aufnahme eines Fensterbauteils, eines Objektivs, eines Stablinsensystems, einer Kamera, eines geordneten Bündels von Lichtleitfasern und/oder anderer Einrichtungen zum Übertragen von Beobachtungslicht und eines Bilds oder eines Bildsignals vom distalen Ende zum proximalen Ende des Endoskops vorgesehen. Der Innenschaft grenzt insbesondere das außerhalb des Innenschafts liegende Lumen für die Lichtleitfasern zum Übertragen von Beleuchtungslicht von dem innerhalb des Innenschafts angeordneten Beobachtungsstrahlengang ab.

Der Innenschaft kann konzentrisch zum Außenschaft angeordnet sein, so dass das Lumen für die Lichtleitfasern beispielsweise einen kreisringförmigen Querschnitt aufweist. Alternativ kann der Innenschaft beispielsweise so im Außenschaft angeordnet sein, dass der Innenschaft den Außenschaft in einem Punkt oder in einer sich vom proximalen Ende bis zum distalen Ende erstreckenden Linie berühren.

Beim Verengen des Lumens wird zumindest der Außenschaft am distalen Ende des Endoskops verformt. Beispielsweise wird der Querschnitt des Außenschafts verringert.

Bei einem Verfahren, wie es hier beschrieben ist, umfasst das Verengen des Lumens insbesondere ein Verschieben zweier das Lumen begrenzenden Wände relativ zueinander. Insbesondere wird das Lumen durch einen Außenschaft und einen Innenschaft begrenzt, von denen wenigstens einer am distalen Ende des Endoskops von einer ideal kreiszylinderförmigen Gestalt abweicht, wobei das Lumen durch Verschieben des Außenschafts relativ zum Innenschaft in einer Richtung parallel oder im Wesentlichen parallel zu einer Symmetrieachse des Außenschafts und/oder des Innenschafts verengt wird.

Bei einem Verfahren, wie es hier beschrieben ist, umfasst das Verengen insbesondere ein Verformen einer das Lumen begrenzenden Wand.

Das Verengen des Lumens umfasst insbesondere ein plastisches und dauerhaftes Verformen einer das Lumen begrenzenden Wand.

Bei einem Verfahren, wie es hier beschrieben ist, umfasst das Verengen insbesondere ein Bördeln oder Schweifen der Wand.

Das Verengen des Lumens umfasst insbesondere ein Bördeln bzw. eine stauchende plastische Verformung eines distalen Randbereichs eines Außenschafts.

Ein Endoskop umfasst Lichtleitfasern zum Leiten von Beleuchtungslicht zum distalen Ende des Endoskops, wobei die Lichtleitfasern in einem Lumen angeordnet sind, und wobei die Lichtleitfasern zumindest an einer Stelle, an der das Lumen bei der Herstellung des Endoskops nach dem Einbringen der Lichtleitfasern in das Lumen verengt wurde, miteinander verschmolzen sind.

Das Endoskop ist insbesondere mittels eines Verfahrens, wie es hier beschrieben ist, hergestellt und weist entsprechende Merkmale, Eigenschaften und Vorteile auf.

### Kurzbeschreibung der Figuren

Nachfolgend werden Ausführungsformen anhand der beigefügten Figuren näher erläutert. Es zeigen:
- Figur 1: eine schematische Darstellung eines Endoskops;
- Figur 2: eine schematische Schnittdarstellung des distalen Endes eines Endoskops;
- Figur 3: eine weitere schematische Schnittdarstellung des distalen Endes des Endoskops aus Figur 2 während seiner Herstellung;
- Figur 4: eine schematische Schnittdarstellung des distalen Endes eines weiteren Endoskops;
- Figur 5: eine weitere schematische Schnittdarstellung des distalen Endes des Endoskops aus Figur 4 während seiner Herstellung;
- Figur 6: eine weitere schematische Schnittdarstellung des distalen Endes des Endoskops aus Figur 4 während seiner Herstellung gemäß einem alternativen Verfahren;
- Figur 7: schematische Darstellungen eines Querschnitts von Lichtleitfasern eines Endoskops;
- Figur 8: ein schematisches Flussdiagramm eines Verfahrens zum Herstellen eines Endoskops.

### Beschreibung der Ausführungsformen

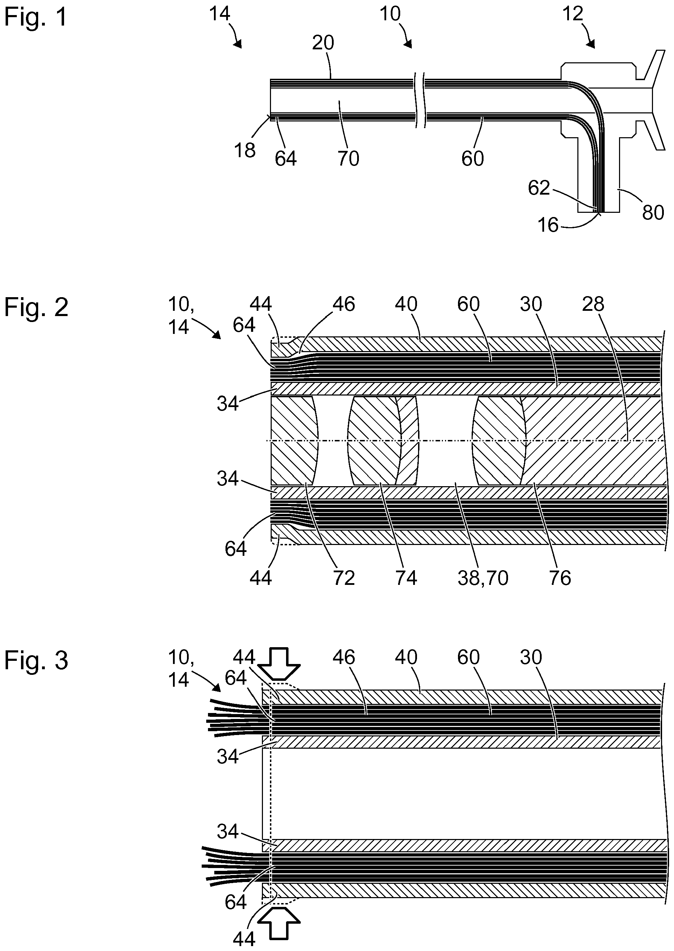

Figur 1 zeigt eine schematische Darstellung eines Endoskops 10 mit einem proximalen Ende 12 und einem distalen Ende 14, zwischen denen sich ein langer Schaft 20 erstreckt. Der Schaft 20 ist insbesondere gerade und starr, kann jedoch alternativ und abweichend von der Darstellung in Figur 1 zumindest abschnittsweise gekrümmt und/oder zumindest abschnittsweise flexibel sein. Im Schaft 20 sind Lichtleitfasern 60 und ein Beobachtungsstrahlengang 70 angeordnet.

Das Endoskop 10 weist am proximalen Ende 12 eine Kupplung 80 zum Koppeln des proximalen Endes 12 des Endoskops 10 mit einer Lichtquelle mittels eines Lichtleitkabels auf. Die externe Lichtquelle und das Lichtleitkabel sind in Figur 1 nicht dargestellt. Ein proximaler Endbereich 62 der Lichtleitfasern 60 ist in der Kupplung 80 angeordnet und weist eine Lichteintrittsfläche 16 für mittels des erwähnten Lichtleitkabels von der erwähnten externen Lichtquelle zu dem Endoskop 10 übertragenes Beleuchtungslicht auf. Ein distaler Endbereich 64 der Lichtleitfasern 60 ist am distalen Ende 14 des Endoskops 10 angeordnet und weist dort, insbesondere an einer distalen Stirnfläche des Schafts 20, eine Lichtaustrittsfläche 18 auf.

Das Endoskop 10 ist insbesondere für medizinische Anwendungen vorgesehen. Zur Sterilisierung soll das Endoskop 10 autoklaviert werden können. Beim Autoklavieren ist das Endoskop 10 über einen Zeitraum von mehreren Minuten bis wenigen Stunden gesättigtem Wasserdampf bei Überdruck und einer Temperatur von ca. 400 Kelvin oder mehr ausgesetzt. In das Endoskop 10 eindringender Wasserdampf oder andere Fluide können das Endoskop 10 beschädigen oder zerstören. Die äußere Oberfläche des Endoskops 10 muss deshalb nicht nur möglichst glatt sein, um ein Anhaften von Verschmutzungen zu verhindern, sondern auch hermetisch dicht, um ein Eindringen von Wasserdampf und anderen Fluiden zu verhindern.

Dies gilt insbesondere auch für die Lichteintrittsfläche 16 am proximalen Ende 12 und die Lichtaustrittsfläche 18 am distalen Ende 14 des Endoskops 10. Nachfolgend ist anhand der Figuren 2 bis 8 dargestellt, wie eine glatte oder weitgehend glatte und insbesondere hermetisch dichte Lichtaustrittsfläche 18 am distalen Ende 14 des Endoskops 10 geschaffen werden kann. Auf ähnliche Weise kann eine weitgehend glatte und insbesondere hermetisch dichte Lichteintrittsfläche 16 am proximalen Ende 12 des Endoskops 10 geschaffen werden.

Figur 2 zeigt eine schematische Schnittdarstellung einer Ausführungsform des distalen Endes 14 eines Endoskops 10, wie es oben anhand der Figur 1 dargestellt ist. Die dargestellte Schnittebene ist parallel zur Zeichenebene der Figur 1 und enthält eine Symmetrieachse 28 des Schafts 20.

Der Schaft 20 umfasst einen Innenschaft 30 und einen Außenschaft 40. Der Innenschaft 30 und der Außenschaft 40 weisen jeweils insbesondere die Gestalt einer Mantelfläche oder eines Ausschnitts einer Mantelfläche eines Kreiszylinders auf und sind koaxial zueinander angeordnet. Im kreisringförmigen Lumen 46 zwischen dem Innenschaft 30 und dem Außenschaft 40 sind die bereits oben anhand der Figur 1 beschriebenen Lichtleitfasern 60 zum Übertragen von Beleuchtungslicht von der Kupplung 80 am proximalen Ende 12 (vgl. Figur 1) zum distalen Ende 14 des Endoskops 10 angeordnet.

Im Lumen 38 des Innenschafts 30 ist ein Beobachtungsstrahlengang 70 zum Übertragen von Licht, das von einem zu beobachtenden Objekt emittiert oder remittiert wird, vom distalen Ende 14 zum proximalen Ende 12 (vgl. Figur 1) des Endoskops 10 angeordnet. Der Beobachtungsstrahlengang umfasst insbesondere ein Lichteintrittsfenster 72, ein Objektiv 74 und mehrere Stablinsen 76, von denen in Figur 2 nur eine teilweise angedeutet ist. Das Lichteintrittsfenster 72 ist insbesondere durch Löten oder auf andere Weise so mit dem distalen Randbereich 34 des Innenschafts 30 gefügt, dass das Lumen 38 des Innenschafts 30 hermetisch dicht verschlossen ist.

Der distale Randbereich 44 des Außenschafts 40 ist durch Bördeln oder auf andere Weise zumindest in umfänglicher Richtung stauchend verformt, so dass das ringförmige Lumen 46 zwischen dem distalen Randbereich 34 des Innenschafts 30 und dem distalen Randbereich 44 des Außenschafts 40 eine reduzierte (in radialer Richtung gemessene) Breite und eine entsprechend reduzierte Querschnittsfläche aufweist. Entsprechend sind die Lichtleitfasern 60 im distalen Endbereich 64 zwischen den distalen Randbereichen 34, 44 des Innenschafts 30 und des Außenschafts 40 komprimiert und verformt. Die Lichtleitfasern 60 sind im distalen Endbereich 64 insbesondere so stark verformt, miteinander verschmolzen bzw. verschweißt und mit den angrenzenden Randbereichen 34, 44 des Innenschafts 30 und des Außenschafts 40 verschweißt, dass sie eine porenarme oder porenfreie und deshalb hermetisch dichte Struktur mit einer glatten Oberfläche, die die Lichtaustrittsfläche 18 (vgl. Figur 1) ist, bildet.

Figur 3 zeigt eine weitere schematische Schnittdarstellung des distalen Endes 14 des Endoskops 10 aus Figur 2. Die Schnittebene der Figur 3 entspricht der Schnittebene der Figur 2. Im Unterschied zu Figur 2 zeigt Figur 3 das distale Ende 14 des Endoskops 10 vor der Verformung des distalen Randbereichs 44 des Außenschafts 40 und der resultierenden Kompression und Verformung der Lichtleitfasern 60.

In der in Figur 3 dargestellten Situation vor der Kompression und Verformung der Lichtleitfasern 60 weisen sowohl der Innenschaft 30 als auch der Außenschaft 40 in ihren distalen Randbereichen 34, 44 jeweils die Gestalt einer Mantelfläche eines Kreiszylinders auf. Nach dem Einbringen der Lichtleitfasern 60 in das ringförmige Lumen 46 zwischen dem Außenschaft 40 und dem Innenschaft 30 wird der distale Randbereich 44 des Außenschafts 40 durch eine gleichzeitig oder nacheinander am gesamten Umfang in radialer Richtung wirkende Kraft, die in Figur 3 durch zwei Pfeile angedeutet ist, plastisch und dauerhaft verformt. Durch diese radial wirkende Kraft wird der distale Randbereich 44 zumindest in umfänglicher Richtung gestaucht bis zu der in Figur 2 angedeuteten Gestalt. Dabei werden die Lichtleitfasern im Bereich zwischen den distalen Randbereichen 34, 44 des Innenschafts 30 und des Außenschafts 40 komprimiert und verformt.

Um eine Verformung der Lichtleitfasern 60, ein Verschmelzen bzw. Verschweißen der Lichtleitfasern 60 untereinander und insbesondere auch ein Verschweißen der Lichtleitfasern 60 mit den distalen Randbereichen 34, 44 von Innenschaft 30 und Außenschaft 40 zu ermöglichen, werden die Lichtleitfasern 60 und insbesondere auch die distalen Randbereiche 34, 44 vor der Verformung auf eine Temperatur erwärmt, bei der die Lichtleitfasern 60 plastisch verformbar sind. Im Fall gläserner Lichtleitfasern liegt diese Temperatur insbesondere innerhalb oder an dem Transformationsbereich, in dem sich die Viskosität des Glases von spröde zu flüssig ändert. Im Fall polymerer Lichtleitfasern 60 liegt die Temperatur insbesondere im Bereich der Glasübergangstemperatur des Polymers oder darüber. Im Fall unterschiedlicher Materialien von Kern und Mantel der einzelnen Lichtleitfasern 60 werden die Lichtleitfasern insbesondere auf eine Temperatur erwärmt, bei der zwar der Kern noch weitgehend unverformbar, jedoch der Mantel bereits plastisch verformbar ist.

Nach dem plastischen Verformen des distalen Randbereichs 44 des Außenschafts 40 und der Lichtleitfasern 60 zwischen den distalen Randbereichen 34, 44 werden die über das distale Ende 14 des Endoskops 10 überstehenden Enden der Lichtleitfasern 60 abgeschnitten. Danach werden die Lichtleitfasern 60 im distalen Endbereich 64 und die distalen Endbereiche 34, 44 von Innenschaft 30 und Außenschaft 40 insbesondere bis zu einer in Figur 3 durch eine gestrichelte Linie angedeuteten Ebene zurückgeschliffen und poliert, um eine glatte und optisch hochwertige Lichtaustrittsfläche 18 der Lichtleitfasern 60 (vgl. Figur 1) zu erzeugen.

Während des Verformens des distalen Randbereichs 44 des Außenschafts 40 und der Lichtleitfasern 60 in ihren zukünftigen distalen Endbereich 64 (vgl. Figur 2) und während des beschriebenen Schleifens und Polierens sind insbesondere das Lichteintrittsfenster 72 und andere Bestandteile des Beobachtungsstrahlungsgangs 70 (vgl. Figur 2) noch nicht in das Lumen 38 des Innenschafts 30 eingesetzt. Stattdessen kann vorübergehend ein Stab entsprechenden Querschnitts in das Lumen 38 des Innenschafts 30 eingesetzt sein, um den distalen Randbereich 34 des Innenschafts 30 zu stützen und eine plastische Verformung des Innenschafts 30 zu verhindern.

Wenn der Außenschaft 40 vor der Verformung auch in seinem zukünftigen distalen Randbereich 44 wie in Figur 3 in durchgezogenen Linien angedeutet die Gestalt einer Mantelfläche eines Kreiszylinders aufweist, weist er nach der beschriebenen plastischen Verformung die in Figur 2 in durchgezogenen Linien angedeutete Gestalt mit einer Stufe in seiner äußeren Oberfläche auf. Um dies zu vermeiden, kann der Außenschaft 40 ursprünglich mit einem in Figur 3 in gestrichelten Linien angedeuteten vergrößerten Querschnitt bzw. mit einer vergrößerten Wandstärke in seinem zukünftigen distalen Randbereich 44 versehen sein. Dies kann - erforderlichenfalls nach einem abschließenden Abdrehen oder Abschleifen - eine glatte und stufenfreie äußere Oberfläche des distalen Randbereichs 44 des Außenschafts 40 ermöglichen. Die Kontur einer solchen glatten und stufenfreien äußeren Oberfläche des distalen Randbereichs ist in Figur 2 durch gestrichelte Linien angedeutet.

Zusätzlich zu der anhand der Figuren 2 und 3 dargestellten, zumindest in Umfangsrichtung stauchenden plastischen Verformung des distalen Randbereichs 44 des Außenschafts 40 kann das Lumen 46 zwischen den distalen Randbereichen 34, 44 von Innenschaft 30 und Außenschaft 40 durch ein Schweifen oder eine andere, zumindest in Umfangsrichtung dehnende plastische Verformung des distalen Randbereichs 34 des Innenschafts 30 verengt werden. Dazu wird beispielsweise vor dem Einsetzen der den Strahlengang 70 definierenden optischen Elemente 72, 74, 76 in das Lumen 38 des Innenschafts 30 (vgl. Figur 2) ein Konus mit spitzem Öffnungswinkel in den distalen Randbereich 34 des Innenschafts 30 gedrückt, um diesen aufzuweiten.

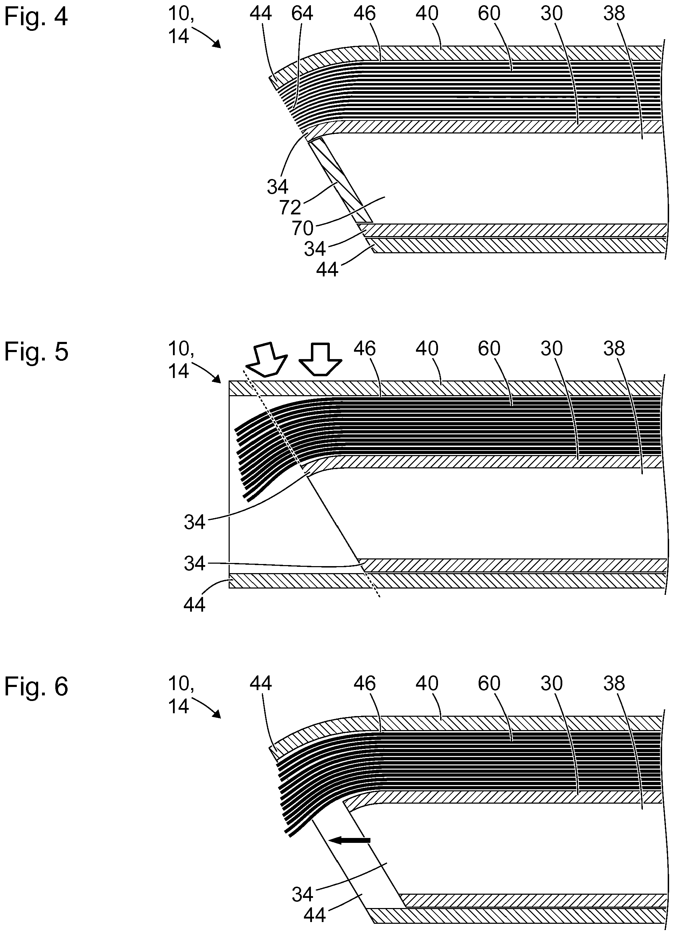

Figur 4 zeigt eine schematische Schnittdarstellung eines distalen Endes 14 eines weiteren Endoskops 10, das in einigen Merkmalen und Eigenschaften den oben anhand der Figuren 1 bis 3 dargestellten Endoskopen ähnelt. Die Schnittebene der Figur 4 entspricht den Schnittebenen der Figuren 2 und 3. Nachfolgend sind Merkmale und Eigenschaften beschrieben, in denen sich das Endoskop 10, dessen distales Ende 14 in Figur 4 dargestellt ist, von den oben anhand der Figuren 1 bis 3 dargestellten Endoskopen unterscheidet.

Das Endoskop 10, dessen distales Ende 14 in Figur 4 dargestellt ist, unterscheidet sich von den oben anhand der Figuren 1 bis 3 dargestellten Endoskopen insbesondere dadurch, dass es nicht für eine gerade Blickrichtung, sondern für einen Winkel von ca. 30 Grad zwischen der Blickrichtung und der Längsachse des Schafts 20 ausgebildet ist. Entsprechend beträgt der Winkel zwischen der Flächennormale eines Lichteintrittsfensters 72 im Beobachtungsstrahlengang 70 und der Längsachse des Schafts 20 ca. 30 Grad. Entsprechend ist auch die Lichtaustrittsfläche 18 geneigt und die Lichtleitfasern 60 nahe der Lichtaustrittsfläche 18 gekrümmt. Insbesondere ist die gesamte Stirnseite des Schafts 20 geneigt.

Das Endoskop 10, dessen distales Ende 14 in Figur 4 dargestellt ist, unterscheidet sich von den oben anhand der Figuren 1 bis 3 dargestellten Endoskopen ferner dadurch, dass der Innenschaft 30 nicht konzentrisch zum Außenschaft 40 angeordnet ist. Stattdessen ist der Innenschaft 30 so im Außenschaft 40 angeordnet, dass beide einander in einer geraden Linie berühren. In diesem linienförmigen Bereich können Innenschaft 30 und Außenschaft 40 durch Löten, Schweißen oder auf andere Weise stoffschlüssig gefügt sein.

Der Innenschaft 30 und der Außenschaft 40 sind in ihren distalen Endbereichen 34, 44 derart gekrümmt, dass nicht nur die erwähnte Krümmung der im Lumen 46 zwischen Innenschaft 30 und Außenschaft 40 angeordneten Lichtleitfasern 60 vorgegeben ist, sondern auch die Querschnittsfläche des Lumens (jeweils bezogen auf Schnittflächen senkrecht zu den Lichtleitfasern 60) nach distal abnimmt. Dadurch sind die Lichtleitfasern 60 in ihrem distalen Endbereich 64 komprimiert.

Indem die Lichtleitfasern 60 vor dem Komprimieren auf eine Temperatur erwärmt wurden, bei der sie zumindest teilweise plastisch verformbar und insbesondere auch miteinander verschmelzbar und mit Innen- und Außenschaft 30, 40 verschweißbar sind, sind die Lichtleitfasern 60 in ihren distalem Endbereich 64 miteinander zumindest teilweise verschmolzen oder verschweißt und mit den distalen Randbereichen 34, 44 von Innenschaft 30 und Außenschaft 40 verschweißt.

Das Verengen des Lumens 46 zwischen den distalen Randbereichen 34, 44 von Innenschaft 30 und Außenschaft 40 kann insbesondere auf zwei verschiedene Weisen erfolgen, die nachfolgend anhand der Figuren 5 dargestellt sind.

Figur 5 zeigt eine weitere schematische Schnittdarstellung des distalen Endes 14 des Endoskops 10 aus Figur 4. Die Schnittebene der Figur 5 entspricht der Schnittebene der Figur 4. Das distale Ende 14 des Endoskops 10 ist in Figur 5 in einer Situation bzw. Konfiguration vor der Fertigstellung des Endoskops 10 und insbesondere vor dem Verengen des Lumens 46 zwischen den distalen Randbereichen 34, 44 von Innenschaft 30 und Außenschaft 40 dargestellt.

Der Außenschaft 40 weist ursprünglich die in Figur 5 angedeutete Gestalt einer Mantelfläche eines Kreiszylinders auf. Der Innenschaft 30 wird in den Außenschaft 40 eingebracht und dort insbesondere befestigt. Die Lichtleitfasern 60 werden in das Lumen 46 zwischen Außenschaft 40 und Innenschaft 30 eingebracht. Danach wird der Außenschaft 40, wie durch zwei Pfeile in Figur 5 angedeutet, plastisch verformt, bis er die in Figur 4 dargestellte Gestalt aufweist. Dazu wird der Außenschaft 40 in seinen zukünftigen distalen Randbereich 44 insbesondere zumindest in Umfangsrichtung gestaucht. Dabei wird das Lumen 46 zwischen Außenschaft 40 und Innenschaft 30 so weit verengt, dass die Lichtleitfasern 60 komprimiert werden und bei einer zuvor eingestellten ausreichend hohen Temperatur auch miteinander zumindest teilweise verschmelzen oder verschweißen und mit den distalen Randbereichen 34, 44 von Innenschaft 30 und Außenschaft 40 verschweißen. Abschließend werden überstehende Bereiche des Außenschafts 40 und der Lichtleitfasern 60 abgeschnitten und bis zu der in Figur 5 durch eine gestrichelte Linie angedeuteten Ebene abgeschliffen und poliert, um eine optisch hochwertige Lichtaustrittsfläche 18 (vgl. Figur 4) zu schaffen.

Figur 6 zeigt das distale Ende 14 des Endoskops 10 aus Figur 4 vor der Fertigstellung bei einem Verfahren, das eine Alternative zu dem oben anhand der Figur 5 dargestellten Verfahren darstellt. Die Schnittebene der Figur 6 entspricht den Schnittebenen der Figuren 2 bis 5.

Im Unterschied zu dem anhand der Figur 5 dargestellten Verfahren weist nicht nur der Innenschaft 30, sondern auch der Außenschaft 40 bereits die endgültige vorgesehene Gestalt auf, bevor die Lichtleitfasern 60 in das Lumen 46 zwischen Außenschaft 40 und Innenschaft 30 eingebracht werden. Der Innenschaft 30 ist jedoch zunächst und in der in Figur 6 dargestellten Situation noch relativ zum Außenschaft 40 nach proximal verschoben. Erst nach dem vollständigen Einbringen der Lichtleitfasern 60 in das Lumen 46 zwischen Außenschaft 40 und Innenschaft 30 wird der Innenschaft 30 relativ zum Außenschaft 40, wie in Figur 6 durch einen Pfeil angedeutet, nach distal verschoben, bis die vorgesehene und in Figur 4 dargestellte relative Position von Innenschaft 30 und Außenschaft 40 erreicht ist. Dadurch wird das Lumen 46 zwischen den distalen Randbereichen 34, 44 von Innenschaft 30 und Außenschaft 40 verengt. Wenn während des Verschiebens des Innenschafts 30 relativ zum Außenschaft 40 und des Verengens des Lumens 46 zwischen deren distalen Randbereichen 34, 44 die Lichtleitfasern eine ausreichende Temperatur aufweisen, verschmelzen oder verschweißen diese zumindest teilweise miteinander und verschweißen zumindest teilweise mit den distalen Randbereichen 34, 44 von Innenschaft 30 und Außenschaft 40.

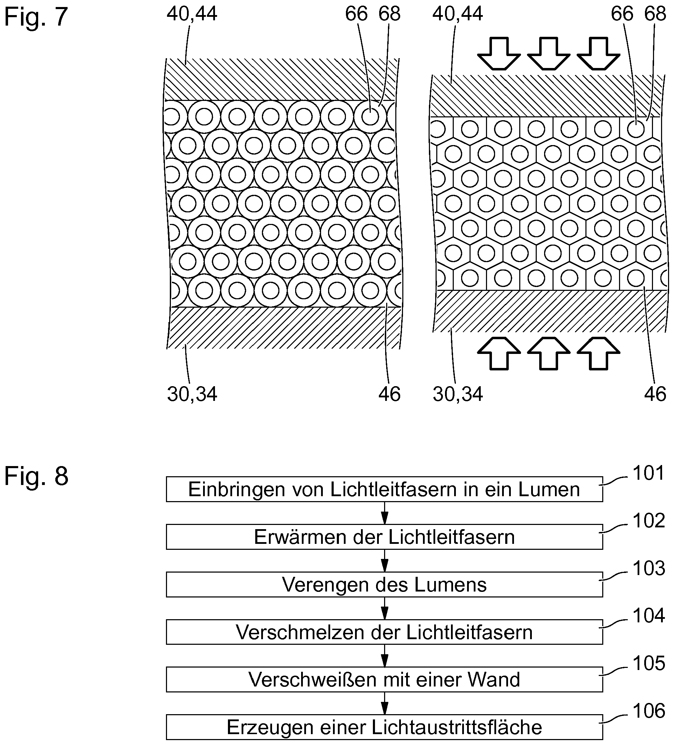

Figur 7 zeigt zwei schematische Schnittdarstellungen von Ausschnitten des Lumens 46 zwischen den distalen Randbereichen 34, 44 von Innenschaft 30 und Außenschaft 40 vor (in Figur 7: links) und nach (in Figur 7: rechts) dem Verengen des Lumens 46. Die Schnittebene ist orthogonal zu den Schnittebenen der Figuren 2 bis 6 und orthogonal zu den Lichtleitfasern 66, 68.

Jede Lichtleitfaser weist einen Kern 66 und einen Mantel 68 aus Materialien unterschiedlicher Brechungsindizes auf. Da der Kern 66 einen höheren Brechungsindex aufweist als der Mantel 68, wird Licht durch Totalreflexion im Kern 66 der Lichtleitfaser 60 geleitet. Vor dem Verengen des Lumens 46 und der Kompression der Lichtleitfasern 60 (in Figur 7: links) weisen sowohl der Kern 66 als auch der Mantel 68 jeder Lichtleitfaser 60 eine im Wesentlichen kreiszylindrische Gestalt auf.

Nach dem Verengen des Lumens 46 und der Verformung der Lichtleitfasern 60 (in Figur 7: rechts) aufgrund der durch Pfeile angedeuteten Kräfte auf die Randbereiche 34, 44 von Innenschaft 30 und Außenschaft 40 weisen die Lichtleitfasern 60 im Idealfall die in Figur 7 rechts dargestellte Gestalt auf. Die äußeren Konturen der Querschnitte der Mäntel 68 der Lichtleitfasern 60 sind sechseckig, grenzen unmittelbar aneinander an und sind insbesondere miteinander verschmolzen oder verschweißt. Soweit die Lichtleitfasern 60 an die distalen Randbereiche 34, 44 von Innenschaft 30 und Außenschaft 40 angrenzen, weisen sie fünfeckige Querschnitte auf und sind mit den distalen Randbereichen 34, 44 von Innenschaft 30 und Außenschaft 40 verschweißt. Die Lichtleitfasern 60 bilden zusammen mit den Randbereichen 34, 44 von Innenschaft 30 und Außenschaft 40 eine starre mechanische Einheit.

Im in Figur 7 rechts dargestellten Idealfall sind die vor dem Verengen des Lumens 46 zwischen den distalen Randbereichen 34, 44 von Innenschaft 30 und Außenschaft 40 (in Figur 7: links) vorliegenden Zwischenräume zwischen den Lichtleitfasern 60 vollständig verschwunden. Die Struktur aus Lichtleitfasern 60 und distalen Randbereichen 34, 44 von Innenschaft 30 und Außenschaft 40 ist hermetisch dicht.

Wenn abweichend von diesem Idealfall Zwischenräume oder Poren verbleiben, beispielsweise aufgrund einer nicht ideal wabenförmigen Anordnung der Querschnitte der Lichtleitfasern 60, können diese Hohlräume nachträglich mit einem Füllmaterial gefüllt und verschlossen werden. Das Füllmaterial wird insbesondere in flüssigem Zustand in die Hohlräume eingebracht und härtet dort beispielsweise aufgrund einer Polymerisation aus. Alternativ wird beispielsweise ein Füllmaterial, dessen Schmelztemperatur niedriger ist als die Schmelztemperatur der Materialien von Lichtleitfasern 60, Innenschaft 30 und Außenschaft 40 in geschmolzenem Zustand in die Hohlräume eingebracht. Als Füllmaterial kann Glaslot, können aber auch andere geeignete Werk- bzw. Füllstoffe dienen.

In dem in Figur 7 rechts dargestellten Idealfall weisen die Kerne 66 der Lichtleitfasern 60 auch nach der Kompression und Verformung der Mäntel 68 kreisförmige Querschnitte auf. Kreisförmige Querschnitte der Kerne 66 der Lichtleitfasern 60 auch nach dem Verengen des Lumens 46 und dem Verformen der Lichtleitfasern 60 können beispielsweise erhalten werden, wenn das Material der Kerne 66 bei der während des Verengens des Lumens 46 gewählten Temperatur eine geringere Plastizität aufweist als das Material der Mäntel 68.

Figur 8 zeigt ein schematisches Flussdiagramm eines Verfahrens zum Herstellen eines Endoskops. Das Verfahren ist auch zum Herstellen eines Endoskops geeignet, das Merkmale und Eigenschaften aufweist, die von den oben anhand der Figuren 1 bis 7 dargestellten abweichen. Trotzdem werden nachfolgend Bezugszeichen aus den Figuren 1 bis 7 beispielhaft verwendet, um das Verständnis des Verfahrens zu vereinfachen.

Bei einem ersten Schritt 101 werden Lichtleitfasern 60 in ein Lumen im zukünftigen Endoskop 10 eingebracht. Das Lumen ist insbesondere ein Zwischenraum zwischen einem Außenschaft 40 und einem Innenschaft 30, der kreisringförmig sein oder eine beliebige andere Gestalt aufweisen kann.

Bei einem zweiten Schritt 102 werden die Lichtleitfasern 60 und mit ihnen insbesondere an die Lichtleitfasern angrenzende Bereiche 34, 44 des zukünftigen Endoskops 10 auf eine Temperatur erwärmt, bei der die Lichtleitfasern 60 zumindest teilweise plastisch verformbar, miteinander verschmolzen oder verschweißt und mit der das Lumen 46 begrenzenden Wand oder den das Lumen 46 begrenzenden Wänden 34, 44 verschweißt werden können.

Bei einem dritten Schritt 103 wird das Lumen 46 verengt. Dies erfolgt insbesondere durch plastisches Verformen zumindest einer das Lumen 46 begrenzenden Wand 34, 44 und/oder durch Verschieben, Schwenken oder Rotieren zweier das Lumen 46 begrenzender Wände 34, 44 relativ zueinander.

Bei einem vierten Schritt 104 werden die Lichtleitfasern 60 zumindest teilweise miteinander verschmolzen oder verschweißt. Bei einem fünften Schritt 105 werden die Lichtleitfasern 60 zumindest teilweise mit einer das Lumen 46 begrenzenden Wand 34, 44 verschweißt. Der vierte Schritt 104 und der fünfte Schritt 105 werden insbesondere zumindest teilweise während des Verengens 103 des Lumens 46 ausgeführt und durch das Verengen 103 des Lumens 46 hervorgerufen.

Bei einem sechsten Schritt 106 wird durch Schleifen und Polieren im Bereich des verengten Lumens 46 eine Lichtaustrittsfläche 18 der Lichtleitfasern 60 erzeugt.

Anhand der Figuren 1 bis 8 wurde primär beschrieben, wie Lichtleitfasern 60 am distalen Ende 14 eines Endoskops 10 durch Verengen eines Lumens 46 komprimiert und miteinander und mit den das Lumen begrenzenden Wänden 34, 44 verschmolzen bzw. verschweißt werden. Auf entsprechende Weise können die Lichtleitfasern 60 in ihren proximalen Endbereichen 62 durch Verengen eines Lumens komprimiert und miteinander verschmolzen oder verschweißt und mit einer das Lumen begrenzenden Wand verschweißt werden. Dazu werden die Lichtleitfasern in ihren zukünftigen proximalen Endbereichen 62 insbesondere in eine metallische Hülse eingebracht und erwärmt. Danach wird die Hülse zumindest lokal durch plastische Verformung verengt. Dadurch werden auch das Lumen der Hülse, in dem die Lichtleitfasern 60 angeordnet sind, verengt und die Lichtleitfasern 60 miteinander und mit der Hülse verschmolzen bzw. verschweißt. Es entsteht eine mechanisch starre Struktur von Lichtleitfasern und Hülse, die in dem oben anhand der Figur 7 dargestellten Idealfall hermetisch dicht sein kann, um das Innere des Endoskops 10 vor dem Eindringen von Wasserdampf und anderen Fluiden zu schützen.

### Bezugszeichen

- 10: Endoskop
- 12: proximales Ende des Endoskops 10
- 14: distale Ende des Endoskops 10
- 16: Lichteintrittsfläche für Beleuchtungslicht am proximalen Ende 12 des Endoskops 10
- 18: Lichtaustrittsfläche für Beleuchtungslicht am distalen Ende 14 des Endoskops 10
- 20: Schaft des Endoskops 10
- 24: distales Ende des Schafts 20
- 28: Symmetrieachse des Schafts 20
- 30: Innenschaft des Endoskops 10
- 34: distaler Randbereich des Innenschafts 30
- 38: Lumen des Innenschafts 30
- 40: Außenschaft des Endoskops 10
- 44: distaler Randbereich des Außenschafts 40
- 46: Lumen zwischen Außenschaft 40 und Innenschaft 30
- 60: Lichtleitfaser
- 62: proximaler Endbereich der Lichtleitfaser 60
- 64: distaler Endbereich der Lichtleitfaser 60
- 66: Kern der Lichtleitfaser 60
- 68: Mantel der Lichtleitfaser 60
- 70: Beobachtungsstrahlengang
- 72: Lichteintrittsfenster im Beobachtungsstrahlengang 70
- 74: Objektiv im Beobachtungsstrahlengang 70
- 76: Stablinse im Beobachtungsstrahlengang 70
- 80: Kupplung zum Koppeln des proximalen Endes 12 des Endoskops 10 mit einer Lichtquelle mittels eines Lichtleitkabels
- 101: erster Schritt (Einbringen von Lichtleitfasern in eine Lumen)
- 102: zweiter Schritt (Erwärmen der Lichtleitfasern)
- 103: dritter Schritt (Verengen des Lumens)
- 104: vierter Schritt (Verschmelzen der Lichtleitfasern)
- 105: fünfter Schritt (Verschweißen der Lichtleitfasern mit einer das Lumen begrenzenden Wand)
- 106: sechster Schritt (Erzeugen einer Lichtaustrittsfläche)

## Patentansprüche

1. **Verfahren zum Herstellen eines Endoskops** (10), mit folgenden Schritten:
**Einbringen** (101) von Lichtleitfasern (60) in ein Lumen (46) in dem Endoskop (10);
**Erwärmen** (102) der Lichtleitfasern (60);
**Verengen** (103) des Lumens (46);
**Verschmelzen** (104) der Lichtleitfasern (60),
wobei das Verengen (103) des Lumens (46) und das Verschmelzen (104) der Lichtleitfasern (60) zumindest teilweise **gleichzeitig** erfolgen.

2. Verfahren nach dem vorangehenden Anspruch, bei dem das Verengen (103) des Lumens (46) und das Verschmelzen (104) der Lichtleitfasern (60) **am distalen Ende** (14) des Endoskops (10) stattfinden.

3. Verfahren nach dem vorangehenden Anspruch, ferner mit folgendem Schritt:
Bilden (106) einer **Lichteintrittsfläche** (16) oder einer **Lichtaustrittsfläche** (18) an den Lichtleitfasern (60) in oder an dem verschmolzenen Bereich (64) der Lichtleitfasern (60).

4. Verfahren nach einem der vorangehenden Ansprüche, bei dem Lichtleitfasern (60) mit einer das Lumen (46) begrenzenden Wand (30, 40) **verschweißt** (105) werden.

5. Verfahren nach einem der vorangehenden Ansprüche, bei dem die Lichtleitfasern (60) **mit einem Außenschaft** (40) des Endoskops (10) verschweißt werden.

6. Verfahren nach einem der vorangehenden Ansprüche, bei dem die Lichtleitfasern (60) **mit einem Innenschaft** (30) des Endoskops (10) verschweißt werden.

7. Verfahren nach einem der vorangehenden Ansprüche, bei dem das Verengen (103) ein **Verschieben** zweier das Lumen (46) begrenzender Wände (30, 40) relativ zueinander umfasst.

8. Verfahren nach einem der vorangehenden Ansprüche, bei dem das Verengen (103) ein **Verformen** einer das Lumen (46) begrenzenden Wand (30, 40) umfasst.

9. Verfahren nach dem vorangehenden Anspruch bei dem das Verengen (103) ein **Bördeln** oder **Schweifen** der Wand (30, 40) umfasst.

10. Endoskop (10) mit einem proximalen Ende (12) und einem distalen Ende (14), zwischen denen sich ein langer Schaft (20) erstreckt,
der einen Innenschaft (30) und einen Außenschaft (40) umfasst,
wobei der Innenschaft (30) und der Außenschaft (40) jeweils insbesondere die Gestalt einer Mantelfläche oder eines Ausschnitts einer Mantelfläche eines Kreiszylinders aufweisen und koaxial zueinander angeordnet sind, wobei im kreisförmigen Lumen zwischen dem Innenschaft (30) und dem Außenschaft (40) Lichtleitfasern (60) zum Übertragen von Beleuchtungslicht angeordnet sind, wobei ein distaler Randbereich (44) des Außenschafts (40) durch Bördeln oder auf andere Weise zumindest in umfänglicher Richtung stauchend verformt ist,
so dass das Lumen (46) zwischen einem distalen Randbereich (34) des Innenschafts (30) und einem distalen Randbereich (44) des Außenschafts (40) eine reduzierte in radialer Richtung gemessene Breite und eine entsprechend reduzierte Querschnittsfläche aufweist, und die Lichtleitfasern (60) im distalen Endbereich (64) zwischen den distalen Randbereichen (34, 44) des Innenschafts (30) und des Außenschafts (40) miteinander verschmolzen sind.

## Claims

1. Method for production of an endoscope (10), with the following steps:
introduction (101) of optical fibres (60) into a lumen (46) in the endoscope (10);
heating (102) of the optical fibres (60);
narrowing (103) of the lumen (46);
melting together (104) of the optical fibres (60),
wherein the narrowing (103) of the lumen (46) and the melting together (104) of the optical fibres (60) take place at least partially at the same time.

2. Method according to the preceding claim, in which the narrowing (103) of the lumen (46) and the melting together (104) of the optical fibres (60) take place at the distal end (14) of the endoscope (10) .

3. Method according to the preceding claim, also with the following step:
formation (106) of a light admission face (16) or of a light exit face (18) on the optical fibres (60) in or on the molten area (64) of the optical fibres (60).

4. Method according to one of the preceding claims, in which optical fibres (60) are welded (105) to a wall (30, 40) delimiting the lumen (46).

5. Method according to one of the preceding claims, in which the optical fibres (60) are welded to an outer shank (40) of the endoscope (10).

6. Method according to one of the preceding claims, in which the optical fibres (60) are welded to an inner shank (30) of the endoscope (10).

7. Method according to one of the preceding claims, in which the narrowing (103) involves two walls (30, 40), which delimit the lumen (46), being moved relative to each other.

8. Method according to one of the preceding claims, in which the narrowing (103) involves a deformation of a wall (30, 40) that delimits the lumen (46).

9. Method according to the preceding claim, in which the narrowing (103) involves a crimping or curving of the wall (30, 40).

10. Endoscope (10) with a proximal end (12) and a distal end (14), between which there extends a long shank (20),
which comprises an inner shank (30) and an outer shank (40),
wherein the inner shank (30) and the outer shank (40) each have in particular the form of a circumferential surface or a portion of a circumferential surface of a circular cylinder and are arranged coaxially with respect to each other, wherein optical fibres (60) for transmitting illumination light are arranged in the circular lumen between the inner shank (30) and the outer shank (40),
wherein a distal edge area (44) of the outer shank (40) is compressively deformed by crimping or other means, at least in the circumferential direction,
such that the lumen (46) between a distal edge area (34) of the inner shank (30) and a distal edge area (44) of the outer shank (40) has a reduced width, measured in the radial direction, and a correspondingly reduced cross-sectional area, and
the optical fibres (60) in the distal end area (64) between the distal edge areas (34, 44) of the inner shank (30) and of the outer shank (40) are melted together.

## Revendications

1. Procédé de fabrication d'un endoscope (10), comprenant les étapes suivantes:
introduire (101) des fibres optiques (60) dans une lumière (46) dans l'endoscope (10);
chauffer (102) les fibres optiques (60);
rétrécir (103) la lumière (46);
fondre (104) les fibres optiques (60),
dans lequel on effectue le rétrécissement (103) de la lumière (46) et la fusion (104) des fibres optiques (60) au moins en partie simultanément.

2. Procédé selon la revendication précédente, dans lequel le rétrécissement (103) de la lumière (46) et la fusion (104) des fibres optiques (60) se produisent à l'extrémité distale (14) de l'endoscope (10).

3. Procédé selon la revendication précédente, comprenant en outre l'étape suivante:
former (106) une face d'entrée de lumière (16) ou une face de sortie de lumière (18) sur les fibres optiques (60) dans ou à la région fondue (64) des fibres optiques (60).

4. Procédé selon l'une quelconque des revendications précédentes, dans lequel on soude (105) les fibres optiques (60) à une paroi (30, 40) limitant la lumière (46) .

5. Procédé selon l'une quelconque des revendications précédentes, dans lequel on soude les fibres optiques (60) à un tube extérieur (40) de l'endoscope (10).

6. Procédé selon l'une quelconque des revendications précédentes, dans lequel on soude les fibres optiques (60) à un tube intérieur (30) de l'endoscope (10).

7. Procédé selon l'une quelconque des revendications précédentes, dans lequel le rétrécissement (103) comprend un déplacement de deux parois (30, 40) limitant la lumière (46) l'une par rapport à l'autre.

8. Procédé selon l'une quelconque des revendications précédentes, dans lequel le rétrécissement (103) comprend une déformation d'une paroi (30, 40) limitant la lumière (46) .

9. Procédé selon l'une quelconque des revendications précédentes, dans lequel le rétrécissement (103) comprend un sertissage ou un soudage de la paroi (30, 40) .

10. Endoscope (10) avec une extrémité proximale (12) et une extrémité distale (14), entre lesquelles s'étend un long tube (20),
qui comprend un tube intérieur (30) et un tube extérieur (40),
dans lequel le tube intérieur (30) et le tube extérieur (40) présentent respectivement en particulier la forme d'une face latérale ou d'une partie d'une face latérale d'un cylindre rond et sont disposés de façon coaxiale l'un à l'autre, dans lequel des fibres optiques (60) sont disposées dans la lumière ronde entre le tube intérieur (30) et le tube extérieur (40) pour la transmission d'une lumière d'éclairage,
dans lequel une région de bord distale (44) du tube extérieur (40) est déformée par sertissage ou d'une autre manière par refoulement au moins en direction périphérique,
de telle manière que la lumière (46) présente entre une région de bord distale (34) du tube intérieur (30) et une région de bord distale (44) du tube extérieur (40) une largeur réduite mesurée en direction radiale et une aire de section transversale réduite de façon correspondante, et que
les fibres optiques (60) soient fondues l'une avec l'autre dans la région d'extrémité distale (64) entre les régions de bord distales (34, 44) du tube intérieur (30) et du tube extérieur (40).
